# EUROPEAN PATENT SPECIFICATION

(11) **EP 4 358 880 B1**
(45) Date of publication and mention of the grant of the patent: **27.05.2026**
(21) Application number: 22743986.6
(22) Date of filing: 17.06.2022
(51) Int. Cl.: A61B 17/80, A61B 17/70, A61B 17/72, A61B 17/00, A61B 17/68

(54) **ADJUSTABLE IMPLANT**
EINSTELLBARES IMPLANTAT
IMPLANT AJUSTABLE

(30) Priority: 25.06.2021 US 202163215105 P
(43) Date of publication of application: 01.05.2024
(73) Proprietor: NuVasive Specialized Orthopedics, Inc., San Diego, CA 92121 (US)
(72) Inventor: LOPEZ CAMACHO, Jorge, San Diego, California 92121 (US)
(74) Representative: Morabito, Sara
(86) International application number: PCT/US2022/033977
(87) International publication number: WO 2022/271550

(56) References cited:
- US-A1- 2011 230 885
- US-A1- 2013 150 889
- US-A1- 2015 230 831
- US-A1- 2020 030 003

## Description

The present patent application claims priority to US Provisional Application No. 63/215,105, filed June 25, 2021.

### TECHNICAL FIELD

The subject matter described herein relates to systems comprising an adjustable implant.

### BACKGROUND

Distraction osteogenesis is a technique which has been used to grow new bone in patients with a variety of defects. For example, limb lengthening is a technique in which the length of a bone (for example a femur or tibia) may be increased. By creating a corticotomy, or osteotomy, in the bone, which is a cut through the bone, the two resulting sections of bone may be moved apart at a particular rate, such as one (1.0) mm per day, allowing new bone to regenerate between the two sections as they move apart. This technique of limb lengthening is used in cases where one limb is longer than the other, such as in a patient whose prior bone break did not heal correctly, or in a patient whose growth plate was diseased or damaged prior to maturity. In some patients, stature lengthening is desired, and is achieved by lengthening both femurs and/or both tibias to increase the patient's height.

Limb lengthening is often performed using external fixation, wherein an external distraction frame is attached to the two sections of bone by pins which pass through the skin. The pins can be sites for infection and are often painful for the patient, as the pin placement or "pin tract" site remains a somewhat open wound throughout the treatment process. The external fixation frames are also bulky, making it difficult for patient to comfortably sit, sleep and move. Intramedullary lengthening devices also exist, such as those described in U.S. Patent Application Publication No. 2011/0060336.
US 2013/150889, US 2020/030003, US 2011/230885 and US 2015/230831 describe adjustable implants know in the art.

### SUMMARY OF THE INVENTION

The invention is defined in the independent claim. Further advantageous embodimentsof the invention are set forth in the dependent claims.

### SUMMARY OF THE DISCLOSURE

It is disclosed an adjustable implant. The adjustable implant may include: a housing configured to be coupled to a first bone portion; an adjustable portion configured to be coupled to a second bone portion, the adjustable portion having a first bar; an actuator rotationally mounted within the housing, the actuator including a protrusion extending therefrom; and at least one gear having an anvil coupled thereto, wherein the protrusion of the actuator is configured to engage the anvil during rotation of the actuator to cause the adjustable portion to move relative to the housing.

It is disclosed an adjustable implant. The adjustable implant may include: a housing configured to be coupled to a first bone portion; an adjustable portion configured to be coupled to a second bone portion, the adjustable portion having a toothed rack; a magnet assembly rotationally mounted within the housing, the magnet assembly including a protrusion extending therefrom, the protrusion having an impact hammer surface; and at least one gear having an anvil coupled thereto, wherein the protrusion of the magnet assembly is configured to engage the anvil during rotation of the magnet assembly, whereby such engagement of the anvil by the impact hammer surface of the protrusion causes the at least one gear to interact with the toothed rack thereby causing the adjustable portion to move relative to the housing.

It is disclosed an adjustable implant. The adjustable implant may include: a housing configured to be coupled to a first bone portion; an adjustable portion configured to be coupled to a second bone portion, the adjustable portion having a toothed rack; a magnet assembly rotationally mounted within the housing, the magnet assembly including a protrusion extending therefrom; a first gear having a first anvil coupled thereto; and a second gear having a second anvil coupled thereto, wherein the protrusion of the magnet assembly is configured to engage the first and second anvils during rotation of the magnet assembly, whereby such engagement of the first and second anvils by the protrusion causes the first and second gears to interact with the toothed rack thereby causing the adjustable portion to move relative to the housing.

It is disclosed but does not fall within the scope of the invention a method of non-invasively adjusting an adjustable implant. The method includes: providing an adjustable implant, the adjustable implant including a housing configured to be coupled to a first bone portion; an adjustable portion configured to be coupled to a second bone portion, the adjustable portion having a first bar; an actuator rotationally mounted within the housing, the actuator including a protrusion extending therefrom; and at least one gear having an anvil coupled thereto; coupling the housing to the first bone portion; coupling the adjustable portion to the second bone portion; and non-invasively adjusting the adjustable implant by causing rotation of the actuator such that the protrusion of the actuator engages with the anvil during rotation of the actuator to cause the adjustable portion to move relative to the housing.

It is also described a system including: an adjustable implant including: a housing configured to be coupled to a first bone portion; an adjustable portion configured to be coupled to a second bone portion, the adjustable portion having a first bar; an actuator rotationally mounted within the housing, the actuator including a protrusion extending therefrom; and at least one gear having an anvil coupled thereto, wherein the protrusion of the actuator is configured to engage the anvil during rotation of the actuator to cause the adjustable portion to move relative to the housing; and an external adjustment device configured to cause actuation of the actuator upon being positioned such that the external adjustment device is perpendicular to the actuator.

It is also described an assembly for an adjustable implant. The assembly includes: an actuator configured to be rotatably mounted within the adjustable implant, the actuator including a protrusion extending therefrom; a first gear having a first anvil coupled thereto; and a second gear having a second anvil coupled thereto, wherein rotation of the actuator causes the protrusion to engage the first anvil and the second anvil to cause the adjustable implant to adjust.

### BRIEF DESCRIPTION OF DRAWINGS

The accompanying drawings, which are incorporated in and constitute a part of this specification, show certain aspects of the subject matter disclosed herein and, together with the description, help explain some of the principles associated with the disclosed implementations. In the drawings,
FIG. 1 shows a perspective view of an adjustable implant according to embodiments of the disclosure;
FIG. 2 shows a perspective cross-sectional view of the adjustable implant of FIG. I;
FIG. 3 shows a perspective view of the actuator according to embodiments of the disclosure;
FIG. 4 shows a cross-sectional view of the adjustable implant in a fully retracted position, according to an embodiment of the disclosure;
FIG. 5 shows a cross-sectional view of the adjustable implant in a first distracted position, according to an embodiment of the disclosure;
FIG. 6 shows a cross-sectional view of the adjustable implant in a second distracted position, according to an embodiment of the disclosure;
FIG. 7 shows a perspective view of a system for non-invasively adjusting a first bone portion and a second bone portion according to embodiments of the disclosure;
FIG. 8 shows a front view of an external adjustment device according to embodiments of the disclosure;
FIG. 9 shows a cross-sectional side view of the external adjustment device of FIG. 8, according to embodiments of the disclosure;
FIG. 10 shows a cross-sectional view of a magnet drive system including a motor having an internal motor speed sensor, according to embodiments of the disclosure;
FIG. 11A shows a magnet of an external adjustment device magnetically coupled to a permanent magnet of an adjustable implant, according to embodiments of the disclosure;
FIG. 11B shows a magnet of the external adjustment device magnetically coupled to the permanent magnet of the adjustable implant, according to embodiments of the disclosure;
FIG. 12 shows a perspective view of a system for non-invasively adjusting a first bone portion and a second bone portion according to aspects of the disclosure;
FIG. 13 shows an exploded view of the housing and adjustable portion of an adjustable implant according to another embodiment of the disclosure;
FIG. 14 shows a cross-sectional view of the adjustable implant of FIG. 13 in a fully retracted position, according to embodiments of the disclosure; and
FIG. 15 shows a cross-sectional view of the adjustable implant of FIG. 13 in a distracted position, according to embodiments of the disclosure.

It is noted that the drawings of the subject matter are not necessarily to scale. The drawings are intended to depict only typical aspects of the subject matter, and therefore, should not be considered as limiting the scope of the disclosed subject matter. In the drawings, like numbering represents like elements between the drawings.

### DETAILED DESCRIPTION

The present disclosure describes various embodiments of an adjustable implant, a system including an adjustable implant and an external adjustment device, and associated methods of adjusting the adjustable implant, the method being not within the scope of the invention. The embodiments described herein can be used in the context of an extramedullary limb lengthening device and system or an intramedullary limb lengthening device and system. It is also contemplated that the embodiments described herein can be used in spinal fixation devices and systems, such as for example, in the treatment of scoliosis or in interspinous process devices. As is described herein, the adjustable implant is driven under impact instead of by a direct drive assembly, resulting in greater distraction for lengthening applications and/or greater retraction force for compression applications (e.g., non- union fractures).

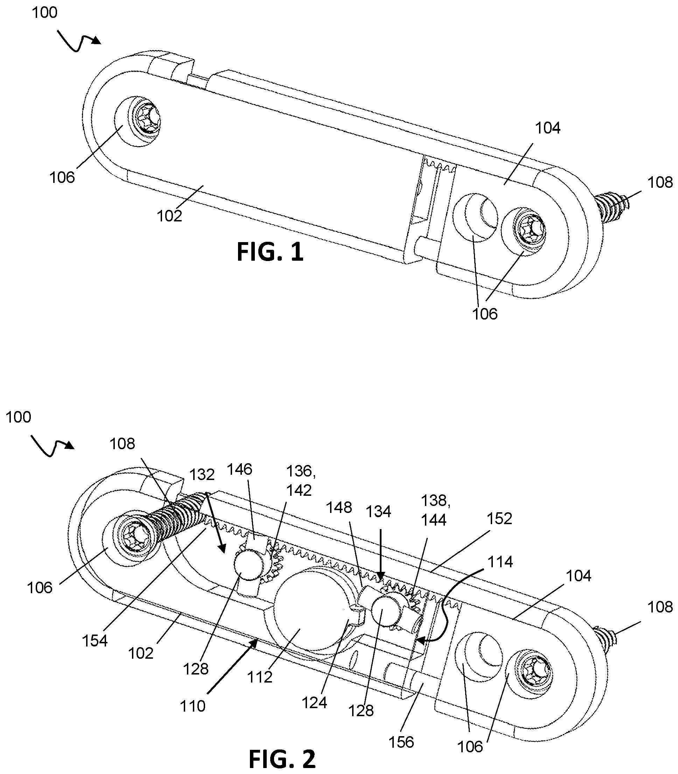

FIGS. 1 and 2 show an adjustable implant 100 according to aspects of the disclosure. The adjustable implant 100 includes a housing 102 configured to be coupled to a first bone portion and an adjustable portion 104 configured to be coupled to a second bone portion. Each of the housing 102 and the adjustable portion 104 can include one or more fixation apertures 106 therein. The fixation apertures 106 may be configured to receive a bone anchor 108 (e.g., a bone fastener, fastener, fixation screw, spinal fixation screw, bone screw, or pedicle screw) therein for securing the adjustable implant 104 to a bone (e.g., a femur, tibia, humerus, one or more pedicles or vertebrae, etc.). The adjustable portion 104 is configured to be moveable relative to the housing 102 to move the second bone portion relative to the first bone portion. Where the movement of the second bone portion is in a direction away from the first bone portion, osteogenesis may occur as will be discussed herein. It is to be understood that the outer profile and/or the overall dimensions (e.g., length, width, height, etc.) of the adjustable implant 100 can be customized based on the desired application of the adjustable implant 100 (i.e., limb lengthening, scoliosis treatment, etc.).

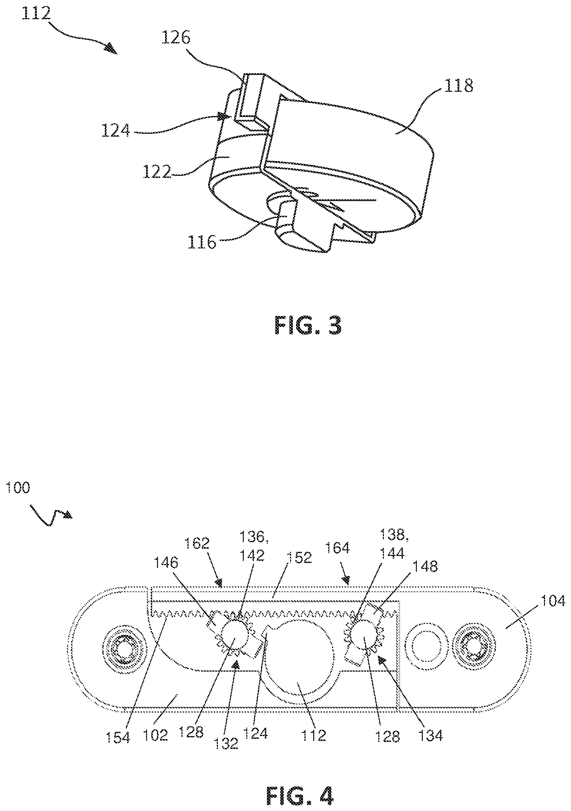

The adjustable implant 100 also includes an assembly 110 (see FIG. 2) for causing adjustment of the adjustable implant. The assembly 110 includes an actuator 112 rotationally mounted within a cavity 114 of the housing 102, for example, via a pin 116 (FIG. 3). However, any other now known or later developed means for rotationally mounting the actuator 112 within the housing 102 can also be used without departing from aspects of the disclosure, such as, for example, a knob, extension, tack, screw, fastener, and/or equivalents thereof. In some embodiments, the actuator 112 can include a stored energy spring, a pressure vessel with relief valve, or a small motor. As shown in FIG. 3, in some embodiments, the actuator 112 can include a magnet 118 and a magnet casing 122. The magnet 118 may include a cylindrical permanent magnet having a north and south pole. The actuator 112 includes a protrusion 124 extending radially therefrom. More specifically, the protrusion 124 can extend from the magnet casing
122. As used herein, "protrusion" may include a bump, knob, jut, projection, prominence, protuberance, overhang, ridge, ledge, and/or equivalents thereof. The protrusion 124 can be integrally formed with the actuator 112 (e.g., the magnet casing 122) and/or fixedly attached thereto. The protrusion 124 can include an impact hammer surface 126. As will be described
in more detail herein, the impact hammer surface 126 acts to provide a large, striking force to cause translation of the adjustable portion 104 relative to the housing 102. The impact hammer surface 126 is composed of a material having sufficient strength and/or hardness to act as an impact hammer without softening the blow each time the impact hammer surface 126 strikes. Examples of such materials include, but are not limited to, chrome plating, nitride-treated steels 64-70 on the Rockwell scale (Re), plasma nitriding, case hardening, and diamond-like coatings (DLC). In other embodiments, the protrusion 124 may include an amorphous metal alloy (liquid metal) insert.

Further, the assembly 110 of the adjustable implant 100 also includes at least one gear assembly rotationally mounted within the cavity 114 of the housing 102, for example, via a pin
128. However, any other now known or later developed means for rotationally mounting the gear assembly within the housing 102 can also be used without departing from aspects of the disclosure, such as, for example, a knob, extension, tack, screw, fastener, and/or equivalents thereof. As shown in FIG. 2, the assembly 110 for the adjustable implant 100 can include two gear assemblies, a first gear assembly 132 and a second gear assembly 134. The two gear assemblies 132, 134 may be positioned on opposing sides of the actuator 112 within the cavity
114. Each gear assembly 132, 134 can include a gear 136, 138 (e.g., a pinion gear) having gear teeth 142, 144 and an anvil 146, 148 coupled thereto. As shown, in some embodiments, the anvil 146, 148 of each gear assembly 132, 134 may extend at least partially through the pin 128, for example, through an aperture formed within the pin 128. However, it is to be understood that any other now known or later developed means for coupling the anvil 146, 148 to the gears 136, 138 can be used without departing from aspects of the disclosure, such as, for example, a tack, weld, bond, joint, and/or equivalents thereof.

As shown in FIGS. 1-2 and 4-6, the adjustable portion 104 includes at least one bar 152. The bar 152 can include, for example, a gear rack having a plurality of teeth 154 extending along a substantial length of the bar 152. However, other configurations are also contemplated by aspects of the disclosure. For example, the bar 152 can include a bar, rod, shaft, rail, or strut with ridges, teeth, threads, or bumps configured to mesh with the gears 136, 138. The bar 152 can be integrally formed with the adjustable portion 104 or fixedly attached thereto. The adjustable portion 104 can include an optional second bar 156 at least partially disposed within the housing 102 for providing additional strength and aid in guiding the movement of the adjustable portion 104 relative to the housing 102. The bar 156 can be configured to move relative to the housing 102 as the adjustable portion 104 moves relative to the housing 102. For example, the bar 156 can slide and/or translate within a second cavity (not shown) within the
housing 102 during movement of the adjustable portion 104. The bar 156 can be integrally formed with the adjustable portion 104 or fixedly attached thereto. In other embodiments, the bar 156 can be replaced with male and female sleeves for providing added guidance and strength.

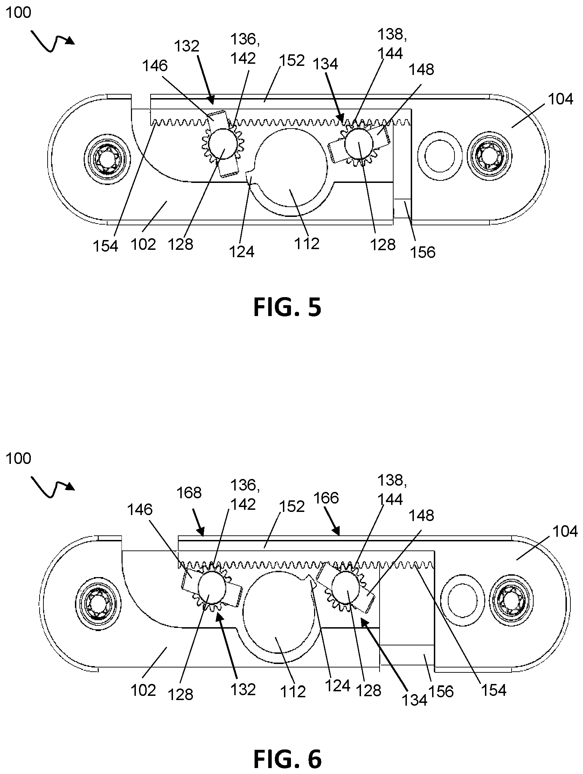

Turning now to FIGS. 4-6, operation of the adjustable implant 100 will now be described. FIG. 4 shows the adjustable implant 100 in a fully retracted state, FIG. 5 shows the adjustable implant 100 in a first distracted state after the anvil 146 has been engaged or stricken by the protrusion 124, and FIG. 6 shows the adjustable implant 100 in a second distracted state after anvil 148 has been engaged or stricken by the protrusion 124.

Upon actuation (i.e., rotation) of the actuator 112, the protrusion 124 of the actuator 112 is configured to engage with the anvil 146 of the first gear assembly 132. More specifically, upon actuation of the actuator 112, the impact hammer surface 126 (FIG. 3) of the protrusion 124 provides a striking force on the anvil 146 of the first gear assembly 132. Such engagement and/or striking force causes the gear 136 to rotate and the gear teeth 142 to mesh with the teeth 154 on the bar 152 at a first location 162 of the bar 152. The meshing of the gear teeth 142 with the teeth 154 of the bar 152 as the gear 136 rotates causes the bar 152 (and thus, the adjustable portion 104) to translate or move relative to the housing 102 to distract the adjustable implant
100. Further, this translation of the bar 152 causes the teeth 154 of the bar 152 at a second location 164 to mesh with the gear teeth 144 of the second gear assembly 134 which in turn positions the anvil 148 of the second gear assembly 134 such that the anvil 148 is in the rotating path of the protrusion 124 and will be engaged by the protrusion 124 during continued rotation of the actuator 112 as shown in FIG. 5.

Upon continued actuation of the actuator 112, the protrusion 124 is configured to engage with the anvil 148 of the second gear assembly 134. More specifically, upon actuation of the actuator 112, the impact hammer surface 126 (FIG. 3) of the protrusion 124 provides a striking force on the anvil 148 of the second gear assembly 134 as shown in FIG. 6. Such engagement and/or striking force causes the gear 138 to rotate and the gear teeth 144 to mesh with the teeth 154 on the bar 152 at a third location 166 of the bar 152. The meshing of the gear teeth 144 with the teeth 154 of the bar 152 as the gear 138 rotates causes the bar 152 (and thus, the adjustable portion 104) to translate or move relative to the housing 102 to distract the adjustable implant 100. Further this translation of the bar 152 causes the teeth 154 of the bar 152 at a fourth location 168 to mesh with the gear teeth 142 of the first gear assembly 132 which in turn positions the anvil 146 of the first gear assembly 132 such that the anvil 146 is in the rotating path of the protrusion 124 and will be engaged by the protrusion 124 during continued rotation of the actuator 112.

While the foregoing describes operation to distract the adjustable implant 100, in some applications, it may be desirable to retract or shorten the adjustable implant 100. The adjustable implant 100 as shown in FIG. 6 can be retracted by causing the actuator 112 to rotate in the opposite way of that described in FIGS. 4-5 such that each strike of the protrusion 124 about anvils 146, 148 causes the gears 136, 138 to rotate in the opposite direction such that as the gear teeth 142, 144 mesh with the bar 152, the bar 152 retracts and moves the adjustable portion 104 toward the housing 102. Retraction may be desirable for non-union fractures.

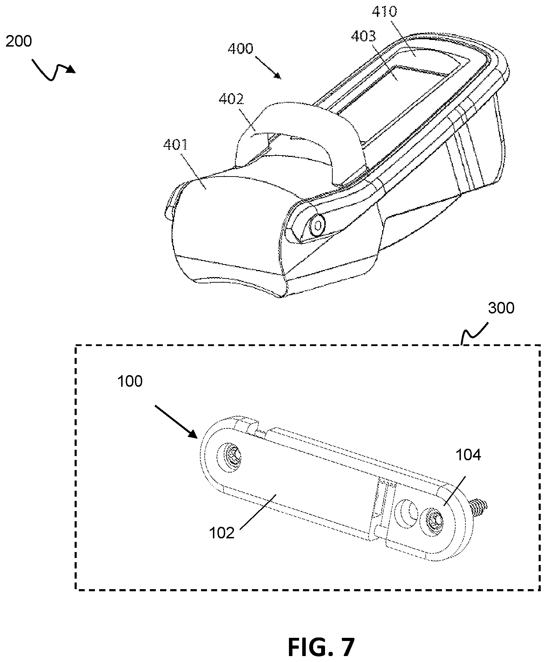

Actuation of the actuator 112 can be caused and controlled by an external adjustment device such as those described in Published International Application No. WO 2020/163800 A1, U.S. Pat. No. 8,382,756, U.S. Pat. No. 9,248,043, U.S. Pat. No. 9,078,711, U.S. Pat. No. 9,044,281, U.S. Pat. No. 11,246,694, U.S. Published Application No. US 2016/0113683 A1, and U.S. Pat. No. 10,835,290 to which reference is made for the relevant parts. Thus, the disclosure also relates to a system 200 (FIG. 7) for adjusting the position of two bone portions relative to each other. The system 200 can include the adjustable implant 100 fixed within a patient 300 and an external adjustment device 400 positioned external to the patient 300. The external adjustment device 400, may include a housing 401 having a handle 402 and a display 403. The handle 402 is shown extending upwardly from the housing 401. In some embodiments, the display 403 may be integrated with the housing 401 of the external adjustment device 400. In the illustrated embodiment, the external adjustment device 400 is configured to receive a removable controller 410 having a display 403, with the display 403 being an integral part of the removable controller 410.

According to an exemplary embodiment, the controller 410 may be a handheld electronic device. The handheld electronic device may be, for example, a smartphone, a tablet, and any other known handheld electronic device. The handheld electronic device may contain and may be operatively connected to a display and/or one or more wireless communication protocols (e.g., Wi-Fi or Bluetooth^{®}). The display of the handheld electronic device may be disposed adjacent to a top surface of the external adjustment device 400, such that the display 403 can communicate information to and receive instructions from a user during use.

For example, in some embodiments the display 403 may present to a user a graphical user interface (GUI). The display 403 may include one or more of a touchscreen or touchscreen technology, including, for example, capacitive touchscreen technology. The GUI may communicate adjustment instructions to a user which may correspond to a treatment regimen to guide the user in adjusting the adjustable implant in accordance with the treatment regimen. Additionally, the GUI may include one or more touchscreen digital buttons configured to activate and control the external adjustment device 400.

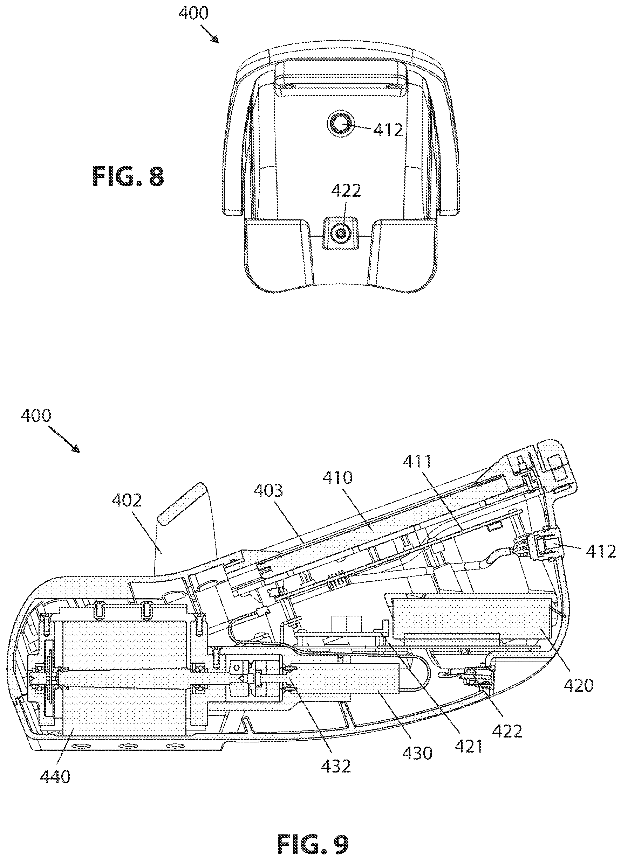

FIG. 8 shows a front view of the external adjustment device 400, the external adjustment device 400 including a power supply input 422 and a data connection port 412. Additionally, a bottom surface 404 of the housing 401 is shown including a curvature configured to form to a patient's body and minimize a distance (gap) between the magnet 440 (FIGS. 9-10 and I IA-1IB) and a magnet 118 (FIG. 3) of the adjustable implant 100 (FIG. 7). The power supply input 422 may be configured to removably receive an AC power supply. The data connection port 412 may be configured to removably receive a data communication cable. The data communication cable may be configured to connect the external adjustment device 400 to a tertiary device to perform one or more of updating the controller 410 software and downloading data from the controller 410.

FIG. 9 shows a cross-sectional side view of the external adjustment device 400 in accordance with the first embodiment. The external adjustment device 400 is shown including the housing 401, the controller 410, an internal power storage device 420, a motor 430, and at least one magnet 440.

The internal power storage device 420 and wireless communication capabilities of the controller 440, may provide for wireless operation of the external adjustment device 400. The internal power storage device 420 may negate the need for a power cord during operation. The controller 410 may provide a low voltage control system negating the need for a bulky external control module. And wireless communication capabilities, for example one or more of radio frequency (RF), Wi-Fi, or Bluetooth^{®} may enable the external adjustment device 400 and the controller 410 to operate remotely. The remote operation may be achieved by one or more of a tertiary device in the same room, and across the internet by a tertiary device on the other side of the globe.

In some embodiments, the controller 410 may be a control board disposed within the housing 401 of the external adjustment device 400. The display 403 may include any type of display 403, including for example: LED, LCD, OLED, and any other known display and touchscreen technology. The control interface board 411 may contain or be in communication with one or more communication circuit, for example, one or more of Wi-Fi, cellular networks, or Bluetooth^{®}, enabling communication between the external adjustment device 400 and one or more tertiary devices.

In FIG. 9, the controller 410 is shown operably connected to a controller interface board 411 by at least one interconnect. In some embodiments, this connection may be established via a physical connection as illustrated, while in other embodiments this connection may be established via a wireless connection, for example, via Bluetooth^{®}. The control interface board 411 may be further connected to one or more of a power interface board 421, the power storage device 420, and the actuator 430.

The controller 410 may be remotely accessible and remotely controllable by a tertiary device allowing for remote operation of the external adjustment device 400 by a user from outside of a sterile field.

The external adjustment device 400 is also shown including an internal power storage device 420. The power storage device 420 may include a battery, a capacitor, and any other power storage device known and used in the art. The power storage device may be rechargeable and the external adjustment device 400 may include a recharging circuit configured to recharge the power storage device 420 using an external power source. The external power source, for example a power supply, may be operably connected to the recharging circuit of the power storage device via the power supply input 422. The power storage device 420, and/or at least a portion of the recharging circuit, may be disposed adjacent to a surface of the external adjustment device 400, enabling connection of a power supply charge cable to the external adjustment device 400. In some embodiments, the recharging circuit may enable wireless charging of the internal power storage device 420, using induction to wirelessly transfer power. In some embodiments, the recharging circuit may be part of and connected to one or more of the power distribution board 421 and the power storage device 400.

In the illustrated embodiment, the power storage device 420 is a battery. The battery 420 is mounted to a chassis of the external adjustment device 400, adjacent to a surface of the external adjustment device 400 enabling connection of a power supply to the external adjustment device 400 at a power supply input 422. The battery 420 includes a power interface board 421, configured to interface with and communicate power to the motor 430. The power interface board 421 may be operably coupled to one or more of the motor 430 and the control interface board 411. The power interface board 421 may also communicate electrical energy from one or more of a power supply input 422 and the power storage device 420, to the controller 410.

The actuator of the external adjustment device 400 includes an electronic motor 430. The driver of the external adjustment device 400 includes a magnet 440 rotatably coupled to the electronic motor 430. The motor 430 may be operably connected to one or more of the
controller 410, the control interface board 411, the power interface board 421, and the internal power storage device 420. In the illustrated embodiment the electronic motor 430 is operably connected to the internal power storage device 420 by the power interface board 421. The power interface board 421 may include power distribution circuits to communicate electrical energy to the electronic motor 430 from one or more of the power supply input 422 and the internal power storage device 420. The power interface board 421 may also be operably connected to the control interface board 411, to relay control information from the controller 410 to the motor 430. In some embodiments, the controller 410 may be in direct communication with the motor 430, and in some embodiments the controller 410 may be connected to the electronic motor via a wireless connection, for example a Bluetooth^{®} connection.

The motor 430 may include any type of motor capable of rotating the magnet 440. The motor 430 is an electric motor and may include a rotational speed sensor 432. The rotational speed sensor 432 may be connected to and in communication with one or more of the control interface board 411 and the controller 410. In some embodiments, the internal speed sensor 432 may include for example one or more of an encoder and a digital output of an electronic motor. In some embodiments, the motor 430 is configured to communicate rotational speed data to the controller 410 wirelessly.

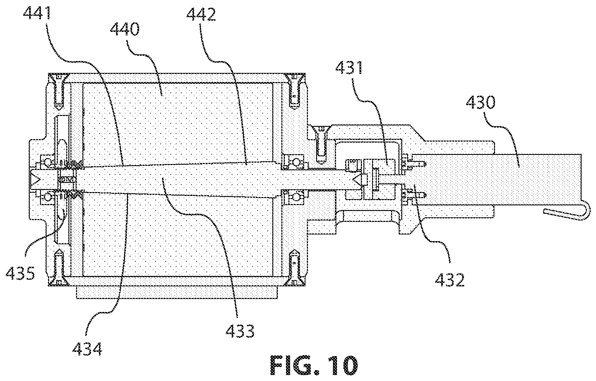

FIG. 10 shows an enhanced cross-sectional view of the motor 430 and the magnet 440 of the external adjustment device 400 (FIG. 9) in accordance with a first embodiment. The magnet 440 is shown rotatably coupled to the motor 430 by one or more couplings 431. In the illustrated embodiment, the magnet 440 includes an internal cavity 441 having an internal surface 442 and having a tapered profile. A magnet drive shaft 433 is shown including a magnet contact surface 434 having a tapered profile. The tapered profile of the magnet drive shaft 433 is configured to communicate with the tapered profile of the internal surface 442 of the magnet 440. This enables the magnet 440 to be secured to the magnet drive shaft 433 by a friction fit. The magnet 440 may be configured to be held onto the magnet drive shaft 433 by a cap 435 and the communicating tapered profiles. In some embodiments, the magnet 440 may be attached to the magnet drive shaft 433 using an adhesive material.

The magnet 440 may comprise any magnetic element including a radially polarized cylindrical magnet, a permanent magnet, an electromagnet, and any other magnetic element known and used in the art. The magnet 440 is configured to magnetically couple with a permanent magnet 118 (FIG. 3) of an adjustable implant 100 (FIG. 7) and to rotate the permanent magnet 118 and adjust the adjustable implant 100. Upon a rotation of the magnet 440, a rotating magnetic field will be generated, placing a force on the magnetically coupled permanent magnet 118 of the adjustable implant 100, thereby inducing a rotation of the permanent magnet 118 and subsequent adjustment of the adjustable implant 100.

In some embodiments, the external adjustment device 400 includes one or more sensors configured to monitor a rotational speed of the magnet 440. In some embodiments, the sensors include magnetic sensors, for example Hall-Effect sensors disposed on one or more of the housing 401 (FIG. 9), a plate, and a chassis, and may be placed adjacent to the magnet 440. In some embodiments, the sensors include photo-sensors. The magnet may include one or more circular optical encoder strips to work in conjunction with the photo-sensors. U.S. Published Application No. US 2016/0113683 A1**,** describes various systems and methods for non-invasively detecting the force generated by a non-invasively adjustable implant.

In the illustrated embodiment, the external adjustment device 400 includes a motor 430 having one or more rotational speed sensor 432 configured to detect a change in a motor angular velocity (V). As described below, sensor 432 may be used to non-invasively detect a rotation of the permanent magnet 118 of the adjustable implant 100. The motor 430 has torque characteristics that allow for little variation in motor angular velocity (V) during a motor rotation and corresponding magnet 440 rotation, when there is no implant or ferrous material located near the ERC magnet or magnetically coupled to the magnet 440.

When an adjustable implant 100 having a magnet 118 is in close proximity to the rotating magnet 440, and is for example magnetically coupled to the magnet 440, the magnetic poles of both magnets cause a changing load on the motor 430 twice per revolution. This causes the magnet 440 to increase or decrease in angular velocity, with the variations detectable by the rotational speed sensor 432.

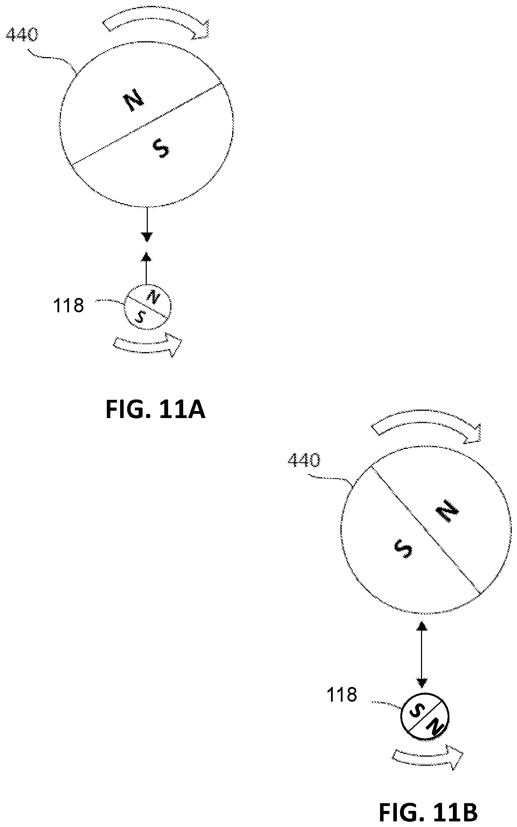

In FIG. 11A, the magnet 440 of the external adjustment device 400 is shown rotating in a first clockwise direction, with the magnet 118 of the adjustable implant 100 (FIG. 7) shown magnetically coupled to the magnet 440 and rotating in a second counterclockwise direction. As one with skill in the art may appreciate, as the motor 430 (FIG. 10) drives rotation of the magnet 440, the respective poles of the magnet 440 and the magnet 118 will attract each other, placing a reduced load on the motor 430 to drive the rotation as the poles are directed towards each other. Comparatively in FIG. 11B, as the motor 430 continues to drive rotation of the magnet 440 the respective poles of the magnet 440 and the permanent magnet 118 will still attract each other, placing an increased load on the motor 430 to drive the rotation as the poles are directed away from each other. These changes in load result in observable changes of the
angular velocity that can be detected by the rotational speed sensor 432 of the motor 430. As it should be appreciated, the rotation of the magnet 440 in one direction results in distraction (or lengthening) of the adjustable implant 100 and rotation of the magnet 440 in the other direction results in retraction (or shortening) of the adjustable implant 100.

The magnet 440 may be configured to rotate at an exemplary speed of 200 revolutions per minute **(RPM)** or greater. This in tum may result in the magnet 118 having a relatively greater rotational speed in RPM, thereby causing the impact hammer force. Specifically, the magnet 118 can rotate at 700 **RPM** or greater to provide the impact hammer force. Referring to FIGS. 11A-11B, as the south pole of the magnet 440 rotates, the north pole of the magnet 118 of the adjustable implant 100 is attracted and pulled. As the south pole of the magnet 440 continues around, the magnet 118 of the adjustable implant 100 is bounced back by the impact reaction forces thereby readying the magnet 118 for another blow.

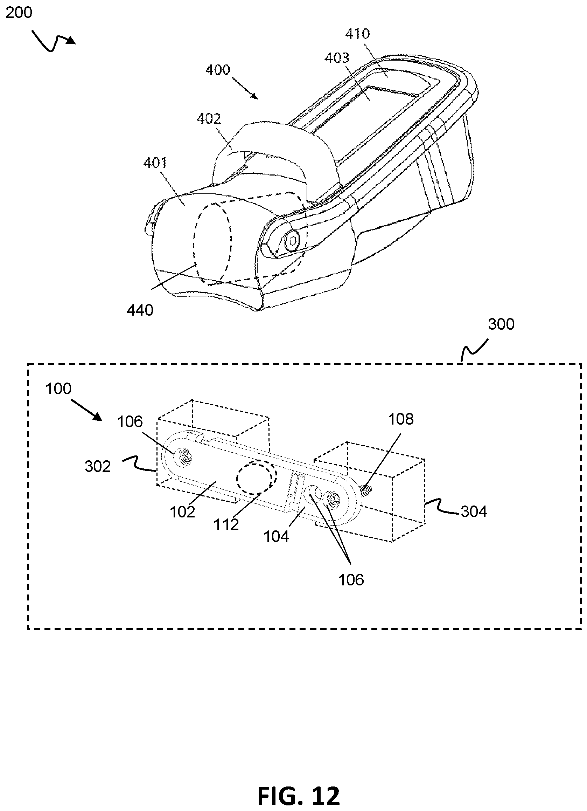

Aspects of the disclosure also include a method for non-invasively adjusting an adjustable implant 100 (the method not being claimed). Referring to FIGS. 2-6 and 12, the method includes providing the adjustable implant 100 having a housing 102 configured to be coupled to a first bone portion 302 (FIG. 12), an adjustable portion 104 configured to be coupled to a second bone portion 304 (FIG. 12), an actuator 112 rotationally mounted within the housing 102 and including a protrusion 124 (FIGS. 2-6) extending therefrom, and at least one gear 136, 138 (FIGS. 2 and
4-6) having an anvil 146, 148 (FIGS. 2 and 4-6) coupled thereto. As shown in FIGS. 2 and 4- 6, the adjustable portion 104 includes at least a first bar 152. The method also includes coupling and/or fixing the housing 102 to the first bone portion 302 and coupling and/or fixing the adjustable portion 104 to the second bone portion 304. The housing 102 and adjustable portion 104 can be coupled/fixed to the respective bone portions 302,304 via bone anchors 108 (FIGS. 1-2 and 12) inserted within fixation apertures 106 (FIGS 1-2) using known techniques such as via creating an operative corridor (e.g., to access the spine, intramedullary canal, or extramedullary location), optionally creating an osteotomy, and driving the bone anchors 108 through the fixation apertures 106 using a driver (not shown). Further, the method includes non-invasively adjusting the adjustable implant 100 by causing rotation of the actuator 112 such that the protrusion 124 (FIGS. 2 and 4-6) of the actuator 112 engages with the anvil 146, 148 during rotation of the actuator 112 to cause the adjustable portion 104 to move relative to the housing 102.

More specifically, the at least one gear can include a first gear 136 having a first anvil 146 coupled thereto and a second gear 138 having a second anvil 148 coupled thereto. During rotation of the actuator 112, the protrusion 124 engages the first anvil 146 to cause the first gear 136 to interact with the first bar 152 at a first location 162 (FIG. 4) on the first bar 152 to cause the adjustable portion 104 to move relative to the housing 102. The interaction of the first gear 138 with the first bar 152 causes the first bar 152 to interact with the second gear 138 at a second location 164 (FIG. 4) on the first bar 152. The interaction of the first bar 152 with the second gear 138 at the second location 164 causes the second anvil 148 to be positioned such that the second anvil 148 shall be engaged by the protrusion 124 during another revolution of the actuator 112 upon continued rotation or due to another treatment session.

After the protrusion 124 engages the first anvil 146, the protrusion 124 engages the second anvil 148 as shown in FIG. 5 to cause the second gear 138 to interact with the first bar 152 to cause the adjustable portion 104 to move relative to the housing 102. The interaction of the second gear 138 with the first bar 152 causes the first bar 152 to interact with the first gear 136 at a third location 168 (FIG. 6) of the first bar 152. The interaction of the first bar 152 with the first gear 136 at the third location 168 causes the first anvil 146 to be positioned such that the first anvil 146 shall be engaged by the protrusion 124 during continued revolution of the actuator 112.

The external adjustment device 400 (FIGS. 7-10 and 12) can be used to non-invasively adjust the adjustable implant 100. Specifically, the external adjustment device 400 can be positioned perpendicularly to the adjustable implant, i.e., the rotating magnet 440 of the external adjustment device 400 can be positioned such that the axis of rotation is perpendicular to the axis of rotation of magnet 118 of the adjustable implant 100. As described herein, the external adjustment device 400 can be positioned against the patient's skin or clothes in proximity to the adjustable implant 100 to provide a magnetic field to the actuator 112 to cause rotation of the actuator 112. As the actuator 112 rotates, the protrusion 124 having an impact hammer surface 126 (FIG. 3) can strike the anvils 146, 148 during rotation to cause translation of the first bar 152, and therefore, the adjustable portion 104 relative to the housing 102. This in turn causes movement of the second bone portion 304 (FIG. 12) relative to the first bone portion 302 (FIG. 12). The impact drive of the adjustable implant 100 provides advantages over direct drive adjustable implants by providing improved force characteristics. The magnet 440 of the external adjustment device 400 is configured to rotate at 200 revolutions per minute (RPM) or greater which in turn results in the actuator 112 having a relatively greater rotational speed in RPM, thereby causing the impact hammer force. Specifically, the actuator 112 can rotate at 700 RPM or greater to provide the impact hammer force. Referring back to FIGS. 11A-11B, as the north pole of the magnet 440 rotates, the north pole of the magnet 118 of the adjustable implant 100 is repelled. As the south pole of the magnet 440 continues around, the magnet 118 of the adjustable implant 100 is pulled back causing a bounce back, thereby readying the magnet 118 for another blow.

Distraction of the adjustable implant 100 can be performed at a desirable rate and duration depending on the desired treatment plan of a medical professional for a particular patient or treatment application. Distraction of the adjustable implant 100 is used to facilitate and control growth of new bone between the two bone portions 302, 304. Retraction of the adjustable implant 100 can be performed in the case of non-union fractures.

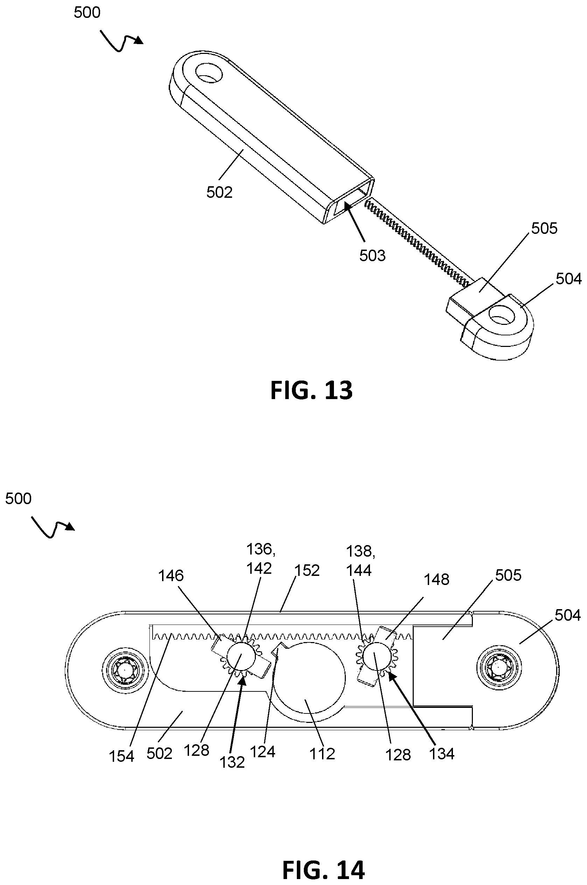

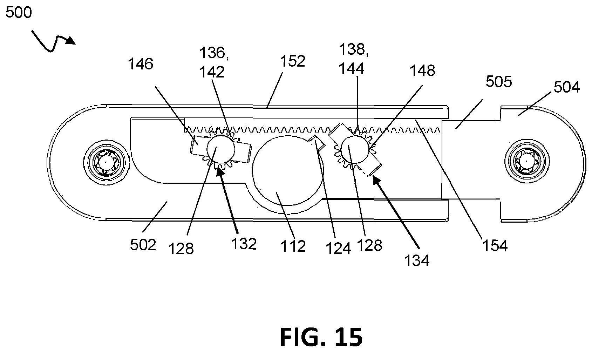

FIGS. 13-14 show an adjustable implant 500 according to another embodiment of the disclosure. In this embodiment, the adjustable implant 500 includes a housing 502 having a cavity 503 (FIG. 13) therein. In this embodiment, the cavity 503 is completely sealed by the housing 502 and a boss 505 of an adjustable portion 504 positioned within the housing 502. The boss 505 can be integrally formed as part of the adjustable portion 504 with the first bar 152. Alternatively, the boss 505 can be a separate component fixed to the adjustable portion 504 and the first bar 152. The boss 505 can be of any complementary shape and/or dimension of the cavity 503 and/or housing 502 such that the cavity 503 remains sealed even during adjustment of the adjustable implant 500. To further aid in sealing, the boss 505 can include a groove in which an o-ring may be positioned and disposed within the groove between the boss 505 and the housing 502. Additionally, the boss 505 can further aid in providing strength and alignment to the adjustable portion 504 as the adjustable portion moves relative to the housing 502, thus, the second bar 156 (FIGS. 2 and 4-6) is not required in this embodiment. The first bar 152 can be wholly positioned within the cavity 503 within the housing 502 in the fully retracted position (FIG. 14) and within a retracted position (FIG. 15). The remainder of the components within the adjustable implant 500 are similar to that of adjustable implant 100 (FIGS. 1-6). Thus, like numbering has been used and duplicate description has been omitted for brevity. Further, the adjustable implant 500 operates in the same way as the adjustable implant 100 (e.g., by non-invasively adjusting the adjustable implant 500 with external adjustment device 400 (FIGS. 7-12)).

In the descriptions above and in the claims, phrases such as "at least one of' or "one or more of' may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, Band C together, or A and Band C together." Use of the term "based on," above and in the claims is intended to mean, "based at least in part on," such that an unrecited feature or element is also permissible.

The terminology used herein is for the purpose of describing particular embodiments only and is not intended to be limiting of the disclosure. As used herein, the terms "first," "second," and the like, do not denote any order, quantity, or importance, but rather are used to distinguish one element from another, and the terms "a" and "an" herein do not denote a limitation of quantity, but rather denote the presence of at least one of the referenced item. It will be further understood that the terms "comprises" and/or comprising," when used in this specification, specify the presence of stated features, integers, steps, operations, elements, and/or components, but do not preclude the presence or addition of one or more other features, integers, steps, operations, elements, components, and/or groups. As used herein, "substantially" refers to largely, for the most part, entirely specified or any slight deviation which provides the same technical benefits of the disclosure.

## Claims

1. A system including:
- an adjustable implant (100) comprising:
a housing (102) configured to be coupled to a first bone portion;
an adjustable portion (104) configured to be coupled to a second bone portion, the adjustable portion (104) having a toothed rack;
a magnet assembly (118) rotationally mounted within the housing (102), the magnet assembly (118) including a protrusion (124) extending therefrom, the protrusion (124) having an impact hammer surface (126); and
at least one gear (132, 134) having an anvil (146, 148) coupled thereto, wherein the protrusion (124) of the magnet assembly (118) is configured to engage the anvil (146, 148) during rotation of the magnet assembly (118) , whereby such engagement of the anvil (146, 148) by the impact hammer surface (126) of the protrusion (124) causes the at least one gear (132, 134) to interact with the toothed rack thereby causing the adjustable portion (104)to move relative to the housing (102)
- and an external adjustment device (400) including
a housing (401),
a controller (410), an internal power storage device (420),
an electronic motor (430) and at least one magnet (440) rotatably coupled to the electronic motor (430) configured to magnetically couple with the permanent magnet (118) of the adjustable implant (100) and to rotate the permanent magnet (118) and adjust the adjustable implant (100) so that upon a rotation of the magnet (440), a rotating magnetic field will be generated, placing a force on the magnetically coupled permanent magnet (118) of the adjustable implant (100), thereby inducing a rotation of the permanent magnet (118) and subsequent adjustment of the adjustable implant (100).

2. The system of claim 1 wherein the external adjustment device (400) includes one or more sensors configured to monitor a rotational speed of the magnet (440).

3. The system of claim 1 or 2, wherein the sensors include magnetic sensors or photo-sensors.

4. The system of any one of the preceding claims, the electronic motor (430) having one or more rotational speed sensor (432) configured to detect a change in a motor angular velocity (V).

5. The system of any one of the preceding claims, wherein the at least one gear (132, 134) includes a first gear (132) having a first anvil (146) and a second gear (134) having a second anvil (148) and wherein preferably the first gear (132) and the second gear (134) are each disposed within the housing (102) on opposing sides of the actuator (112).

6. The system of claim 5, wherein the adjustable portion (104) includes a first bar (152) including a gear rack having a plurality of teeth (154) extending along a substantial length of the first bar (152).

7. The system of claim 6, wherein the adjustable portion (104) includes a second bar (156) at least partially disposed within the housing (102) for providing additional strength and aid in guiding the movement of the adjustable portion (104) relative to the housing (102), the second bar (156) being configured to move relative to the housing (102) as the adjustable portion (104) moves relative to the housing (102).

8. The system of claim 6, wherein the protrusion (124) of the actuator (112) is configured to engage the first anvil (146) of the first gear (132) and the second anvil (148) of the second gear (134) during rotation of the actuator (112), whereby engagement of the first and second anvils (146, 148) by the protrusion (124) causes the first and second gears (132, 134) to interact with the first bar (152) thereby causing the adjustable portion (104) to move relative to the housing (102) and wherein preferably the engagement of the protrusion (124) with the second anvil (148) during rotation of the actuator (112) causes the first bar (152) to interact with the first gear (132) to cause the first anvil (146) to be positioned such that, upon continued rotation of the actuator (112), the protrusion (124) of the actuator (112) engages the first anvil (146) and wherein preferably the engagement of the protrusion (124) with the first anvil (146) during rotation of the actuator (112) causes the first bar (152) to interact with the second gear (134) to cause the second anvil (148) to be positioned such that, upon continued rotation of the actuator (112), the protrusion (124) of the actuator (112) engages the second anvil (148).

9. The adjustable implant (100) of claim 1, wherein the magnet (118; 440) is disposed within a magnet casing (122), wherein the protrusion (124) extends from the magnet casing (122) and wherein the magnet (118; 440) is configured to rotate by application of a non-invasively applied magnetic field and wherein preferably the housing (102) and the adjustable portion (104) each include an aperture for accommodating a fixation screw (108) therein.

## Patentansprüche

1. System, umfassend:
- ein einstellbares Implantat (100), umfassend:
ein Gehäuse (102), das eingerichtet ist, um mit einem ersten Knochenabschnitt verbunden zu werden;
einen einstellbaren Abschnitt (104), der eingerichtet ist, um mit einem zweiten Knochenabschnitt verbunden zu werden, wobei der einstellbare Abschnitt (104) eine Zahnstange aufweist;
eine Magnetanordnung (118), die drehbar innerhalb des Gehäuses (102) montiert ist, wobei die Magnetanordnung (118) einen sich davon erstreckenden Vorsprung (124) umfasst, wobei der Vorsprung (124) eine Schlaghammerfläche (126) aufweist; und
zumindest ein Zahnrad (132, 134) mit einem daran verbundenen Amboss (146, 148), wobei der Vorsprung (124) der Magnetanordnung (118) eingerichtet ist, um mit dem Amboss während einer Drehung der Magnetanordnung (118) in Eingriff zu kommen, wodurch ein derartiger Eingriff des Ambosses (146, 148) mit der Schlaghammerfläche (126) des Vorsprungs (124) bewirkt, dass zumindest ein Zahnrad (132, 134) mit der Zahnstange zusammenwirkt, wodurch ein Bewegen des einstellbaren Abschnitts (104) relativ zum Gehäuse (102) bewirkt wird,
- und eine externe Einstellvorrichtung (400), die ein Gehäuse (401), eine Steuerung (410), eine interne Energiespeichervorrichtung (420), einen Elektromotor (430) und zumindest einen Magneten (440) umfasst, der drehbar mit dem Elektromotor (430) verbunden ist, der eingerichtet ist, um sich magnetisch mit dem Permanentmagneten (118) des einstellbaren Implantats (100) zu verbinden und den Permanentmagneten (118) zu drehen und das einstellbare Implantat (100) einzustellen, so dass bei einer Drehung des Magneten (440) ein rotierendes Magnetfeld erzeugt wird, das eine Kraft auf den magnetisch verbundenen Permanentmagneten (118) des einstellbaren Implantats (100) aufbringt, wodurch eine Drehung des Permanentmagneten (118) und eine anschließende Einstellung des einstellbaren Implantats (100) veranlasst wird.

2. System nach Anspruch 1, wobei die externe Einstellvorrichtung (400) einen oder mehrere Sensoren umfasst, die eingerichtet sind, um eine Drehzahl des Magneten (440) zu überwachen.

3. System nach Anspruch 1 oder 2, wobei die Sensoren Magnetsensoren oder Fotosensoren umfassen.

4. System nach einem der vorhergehenden Ansprüche, wobei der Elektromotor (430) einen oder mehrere Drehzahlsensoren (432) aufweist, die eingerichtet sind, um eine Änderung der Motorwinkelgeschwindigkeit (V) zu erfassen.

5. System nach einem der vorhergehenden Ansprüche, wobei das zumindest ein Zahnrad (132, 134) ein erstes Zahnrad (132) mit einem ersten Amboss (146) und ein zweites Zahnrad (134) mit einem zweiten Amboss (148) umfasst, und wobei vorzugsweise das erste Zahnrad (132) und das zweite Zahnrad (134) jeweils innerhalb des Gehäuses (102) auf gegenüberliegenden Seiten des Aktuators (112) angeordnet sind.

6. System nach Anspruch 5, wobei der einstellbare Abschnitt (104) eine erste Stange (152) mit einer Zahnstange mit einer Mehrzahl von Zähnen (154) umfasst, die sich entlang einer wesentlichen Länge der ersten Stange (152) erstrecken.

7. System nach Anspruch 6, wobei der einstellbare Abschnitt (104) eine zweite Stange (156) umfasst, die zumindest teilweise innerhalb des Gehäuses (102) angeordnet ist, um eine zusätzliche Festigkeit vorzusehen und eine Führung der Bewegung des einstellbaren Abschnitts (104) relativ zum Gehäuse (102) zu unterstützen, wobei die zweite Stange (156) eingerichtet ist, um sich relativ zum Gehäuse (102) zu bewegen, wenn sich der einstellbare Abschnitt (104) relativ zum Gehäuse (102) bewegt.

8. System nach Anspruch 6, wobei der Vorsprung (124) des Aktuators (112) eingerichtet ist, um mit dem ersten Amboss (146) des ersten Zahnrads (132) und dem zweiten Amboss (148) des zweiten Zahnrads (134) während einer Drehung des Aktuators (112) in Eingriff zu kommen, wobei ein Eingriff der ersten und zweiten Ambosse (146, 148) mit dem Vorsprung (124) bewirkt, dass die ersten und zweiten Zahnräder (132, 134) mit der ersten Stange (152) zusammenwirken, wodurch ein Bewegen des einstellbaren Abschnitts (104) relativ zum Gehäuse (102) bewirkt wird, und wobei vorzugsweise der Eingriff des Vorsprungs (124) mit dem zweiten Amboss (148) während einer Drehung des Aktuators (112) bewirkt, dass die erste Stange (152) mit dem ersten Zahnrad (132) zusammenwirkt, um zu bewirken, das der erste Amboss (146) derart positioniert wird, dass bei fortgesetzter Drehung des Aktuators (112) der Vorsprung (124) des Aktuators (112) mit dem ersten Amboss (146) in Eingriff kommt, und wobei vorzugsweise der Eingriff des Vorsprungs (124) mit dem ersten Amboss (146) während einer Drehung des Aktuators (112) bewirkt, dass die erste Stange (152) mit dem zweiten Zahnrad (134) zusammenwirkt, um zu bewirken, das der zweite Amboss (148) derart positioniert wird, dass bei fortgesetzter Drehung des Aktuators (112) der Vorsprung (124) des Aktuators (112) mit dem zweiten Amboss (148) in Eingriff kommt.

9. Einstellbares Implantat (100) nach Anspruch 1, wobei der Magnet (118; 440) in einem Magnetgehäuse (122) angeordnet ist, wobei sich der Vorsprung (124) vom Magnetgehäuse (122) erstreckt und wobei der Magnet (118; 440) eingerichtet ist, um sich durch Anlegen eines nicht-invasiv angelegten Magnetfelds zu drehen, und wobei vorzugsweise das Gehäuse (102) und der einstellbare Abschnitt (104) jeweils eine Öffnung zur Aufnahme einer Befestigungsschraube (108) darin umfassen.

## Revendications

1. Système comprenant :
- un implant réglable (100) comprenant :
un boîtier (102) configuré pour être couplé à une première partie osseuse ;
une partie réglable (104) configurée pour être couplée à une seconde partie osseuse, la partie réglable (104) ayant une crémaillère ;
un ensemble magnétique (118) monté en rotation à l'intérieur du boîtier (102),
l'ensemble magnétique (118) comprenant une saillie (124) s'étendant à partir de celui-ci, la saillie (124) ayant une surface de marteau d'impact (126) ; et
au moins une roue dentée (132, 134) à laquelle est couplée une enclume (146, 148), dans lequel la saillie (124) de l'ensemble magnétique (118) est configurée pour venir en prise avec l'enclume (146, 148) pendant la rotation de l'ensemble magnétique (118), la mise en prise de l'enclume (146, 148) par la surface de marteau d'impact (126) de la saillie (124) entraînant l'interaction de l'au moins une roue dentée (132, 134) avec la crémaillère, provoquant ainsi le déplacement de la partie réglable (104) par rapport au boîtier (102)
- et un dispositif de réglage externe (400) comprenant
un boîtier (401),
un dispositif de commande (410), un dispositif de stockage d'énergie interne (420),
un moteur électronique (430) et au moins un aimant (440) couplé de manière rotative au moteur électronique (430) configuré pour se coupler magnétiquement à l'aimant permanent (118) de l'implant réglable (100) et pour faire tourner l'aimant permanent (118) et régler l'implant réglable (100) de manière à ce que la rotation de l'aimant (440) génère un champ magnétique rotatif qui exerce une force sur l'aimant permanent (118) couplé magnétiquement de l'implant réglable (100), induisant ainsi une rotation de l'aimant permanent (118) et le réglage ultérieur de l'implant réglable (100).

2. Système selon la revendication 1 dans lequel le dispositif de réglage externe (400) comprend un ou plusieurs capteurs configurés pour surveiller la vitesse de rotation de l'aimant (440).

3. Système selon la revendication 1 ou 2, dans lequel les capteurs comprennent des capteurs magnétiques ou des photocapteurs.

4. Système selon l'une quelconque des revendications précédentes, le moteur électronique (430) ayant un ou plusieurs capteurs de vitesse de rotation (432) configurés pour détecter un changement de la vitesse angulaire du moteur (V).

5. Système selon l'une quelconque des revendications précédentes, dans lequel l'au moins une roue dentée (132, 134) comprend une première roue dentée (132) ayant une première enclume (146) et une seconde roue dentée (134) ayant une seconde enclume (148) et dans lequel, de préférence, la première roue dentée (132) et la seconde roue dentée (134) sont chacune disposées à l'intérieur du boîtier (102) sur des côtés opposés de l'actionneur (112).

6. Système selon la revendication 5, dans lequel la partie réglable (104) comprend une première barre (152) comprenant une crémaillère ayant une pluralité de dents (154) s'étendant sur une longueur substantielle de la première barre (152).

7. Système selon la revendication 6, dans lequel la partie réglable (104) comprend une seconde barre (156) au moins partiellement disposée à l'intérieur du boîtier (102) pour fournir une résistance supplémentaire et aider à guider le mouvement de la partie réglable (104) par rapport au boîtier (102), la seconde barre (156) étant configurée pour se déplacer par rapport au boîtier (102) lorsque la partie réglable (104) se déplace par rapport au boîtier (102).

8. Système selon la revendication 6, dans lequel la saillie (124) de l'actionneur (112) est configurée pour venir en prise avec la première enclume (146) de la première roue dentée (132) et la seconde enclume (148) de la seconde roue dentée (134) pendant la rotation de l'actionneur (112), de sorte que la mise en prise des première et seconde enclumes (146, 148) par la saillie (124) provoque l'interaction des première et seconde roues dentées (132, 134) avec la première barre (152), provoquant ainsi le déplacement de la partie réglable (104) par rapport au boîtier (102) et dans lequel, de préférence, la mise en prise de la saillie (124) avec la seconde enclume (148) pendant la rotation de l'actionneur (112) provoque l'interaction de la première barre (152) avec la première roue dentée (132) afin de positionner la première enclume (146) de manière à ce que, lors de la rotation continue de l'actionneur (112), la saillie (124) de l'actionneur (112) vienne en prise avec la première enclume (146) et dans lequel, de préférence, la mise en prise de la saillie (124) avec la première enclume (146) pendant la rotation de l'actionneur (112) provoque l'interaction de la première barre (152) avec la seconde roue dentée (134) afin de positionner la seconde enclume (148) de telle sorte que, lors de la rotation continue de l'actionneur (112), la saillie (124) de l'actionneur (112) vienne en prise avec la seconde enclume (148).

9. Implant réglable (100) selon la revendication 1, dans lequel l'aimant (118 ; 440) est disposé dans un boîtier d'aimant (122), dans lequel la saillie (124) s'étend à partir du boîtier d'aimant (122) et dans lequel l'aimant (118 ; 440) est configuré pour tourner par l'application d'un champ magnétique appliqué de manière non invasive et dans lequel, de préférence, le boîtier (102) et la partie réglable (104) comprennent chacun une ouverture pour loger une vis de fixation (108) à l'intérieur de celle-ci.
